# EUROPÄISCHE PATENTSCHRIFT

(11) **EP 0 949 908 B1**
(45) Veröffentlichungstag und Bekanntmachung des Hinweises auf die Patenterteilung: **30.01.2002**
(21) Anmeldenummer: 97948876.4
(22) Anmeldetag: 04.11.1997
(51) Int. Cl.: A61K 9/20, A61K 9/70

(54) **ZUBEREITUNG, BESTEHEND AUS EINER FLÄCHENHAFTEN, FILM- ODER OBLATENARTIGEN DARREICHUNGSFORM**
PREPARATION CONSISTING OF A SURFACE-ADHERING, FILM-LIKE OR WAFER-LIKE ADMINISTRATION FORM
PREPARATION CONSTITUEE D'UNE FORME D'ADMINISTRATION ADHESIVE EN SURFACE, DU TYPE FILM OU CACHET

(30) Priorität: 13.11.1996 DE 19646836
(43) Veröffentlichungstag der Anmeldung: 20.10.1999
(73) Patentinhaber: LTS LOHMANN Therapie-Systeme AG, 56626 Andernach (DE)
(72) Erfinder: LUDWIG, Karin, D-56589 Datzeroth (DE)
(74) Vertreter: Flaccus, Rolf-Dieter, Dr.
(86) Internationale Anmeldenummer: EP9706082
(87) Internationale Veröffentlichungsnummer: WO9820859

(56) Entgegenhaltungen:
- EP-A- 0 398 229
- WO-A-95/09608
- DATABASE WPI Section Ch, Week 9715 Derwent Publications Ltd., London, GB; Class B07,Page p33, AN 97-154886 XP002055483 & CN 1 091 003 A (YANG, J.) , 24.August 1994

## Beschreibung

Die Erfindung betrifft eine Zubereitung, bestehend aus einer flächenhaften, film- oder oblatenartigen Darreichungsform mit einer Codierung durch Schrift- und/oder graphische Zeichen bzw. Muster zur Kennzeichnung der daraus bei Anwendung freisetzbaren geschmacks- oder geruchswirksamen und/oder pflegenden bzw. heilenden Substanzen.

Immer häufiger sind Hersteller pharmazeutischer Produkte verpflichtet, außer der Primärverpackung auch das Produkt als solches zu kennzeichnen. Dies betrifft insbesondere Medikamente in einer Darreichungsform mit dem Ziel einer kontrollierten Wirkstofffreigabe.
Beim Stand der Technik erfolgt eine Codierung von Medikamenten in Form von Pillen, Tabletten bzw. folien- oder filmartigen Produkten üblicherweise in einem zusätzlichen Arbeitsgang zur eigentlichen Herstellung durch Bedrucken oder Einstanzen von speziellen Kennzeichen oder Mustern. Neben dem dadurch erforderlichen Aufwand mit entsprechend erhöhten Herstellkosten ist beispielsweise beim Bedrucken das Einbringen eines Druckmediums, einer Druckfarbe, nicht unumstritten, weil damit zumindest in einigen Fällen das Risiko einer Allergisierung gegeben bzw. unnötig vergrößert ist.

Der Erfindung liegt die Aufgabe zugrunde, für eine Zubereitung der im gattungsbildenden Oberbegriff bezeichneten Gattung eine Codierung durch Schrift- und/oder graphische Zeichen bzw. Muster anzugeben, welche die vorgenannten Nachteile vermeidet, insbesondere das Aufbringen eines Fremdstoffes wie Druckfarbe, und damit das Risiko der Allergisierung vermeidet und den entsprechenden Aufwand für eine der Herstellung nachgeordnete Codierung reduziert.

Die Lösung der Aufgabe gelingt bei einer Zubereitung der im Oberbegriff von Anspruch 1 genannten Art mit der Erfindung dadurch, daß die Codierung durch Flächenbereiche mit optisch wahrnehmbaren Dickenunterschieden vorgenommen ist, derart, daß dusch die unterschiedlich dichen Flächenbereiche eine in der Durchsicht erkennbare Markierung gegeben ist.

Durch die Ausbildung unterschiedlich dicker Flächenbereiche zwecks Codierung vermeidet man das Aufbringen eines Logos durch Bedrucken mit Lebensmittelfarben und die hierzu beschriebenen Nachteile. Stattdessen wird erfindungsgemäß durch musterförmig verlaufende Unterschiede in der Schichtdicke der film- oder oblatenartigen Darreichungsform ein dem Auge sichtbares Logo im Flächenbereich der Zubereitung erzeugt, das in der Wirkung etwa einem sogenannten Wasserzeichen bei papierartigen Dispersionen entspricht. Die Erfindung stellt demnach eine vorteilhafte Alternative zur Anbringung von Produktkennzeichnungen durch Bedrucken mit Fremdstoffen dar. Zwar beruht die Bildung von "Wasserzeichen" beim Trocknen von Dispersionen auf strukturierten Unterlagen ebenfalls auf der Ausbildung von Dickenschwankungen der flächenhaften papierartigen Dispersionen, jedoch wurde noch niemals vor- geschlagen, diese Art einer Kennzeichnung im Zusammenhang mit film-, papier- oder oblatenartigen Darreichungsformen pflegender oder heilender Stoffe anzuwenden.

Eine Ausgestaltung der Zubereitung sieht vor, daß die Bereiche unterschiedlicher Dicke herstellungsbedingt unter Vermeidung einer unterschiedlichen Dichte bzw. Verdichtung ausgebildet sind.

Eine weitere Ausgestaltung der Zubereitung sieht vor, daß die Bereiche unterschiedlicher Dicke eine unterschiedliche Dichte (Verdichtung) aufweisen.

Das erfindungsgemäße Verfahren zur Herstellung der Zubereitung sieht vor, daß das Einbringen der Zeichen bzw. Muster der Codierung durch eine irreversible Deformation im plastisch verformbaren Zustand erfolgt.

Ein erfindungsgemäßes Verfahren zur Herstellung der Zubereitung sieht vor, daß die Zeichen bzw. Muster der Codierung im Verlauf der Herstellung ohne Einwirkung von Druck (Verdichtung) ausgebildet werden.

Ein weiteres erfindungsgemäßes Verfahren zur Herstellung der Zubereitung sieht vor, daß die Zeichen bzw. Muster der Codierung unter Einwirkung von Druck (Verdichtung) angebracht werden.

Eine Ausgestaltung sieht vor, daß die Dickenunterschiede durch Beschichten einer Unterlage hergestellt werden, die eine der Codierung entsprechende Folge von Erhebungen und/oder Vertiefungen aufweist. Solche Erhebungen bzw. Vertiefungen können beispielsweise auf einer druckplatten-
ähnlichen Unterlage durch Aufbringen einer Radierung oder Ätzung aufgebracht sein.

Nach einem weiteren erfindungsgemäßen Vorschlag können die Dickenunterschiede durch Beaufschlagung der Zubereitung beim Trocknen mit lokalen Temperaturunterschieden hergestellt werden.

Nach einem weiteren erfindungsgemäßen Verfahren zur Herstellung der Zubereitung werden die Dickeunterschiede kontinuierlich, z.B. mit einer strukturierten Prägewalze oder intermittierend, z.B. mit einem Stempel,eingebracht.

Im folgenden wird die Herstellung codierter flächenhafter Darreichungsformen anhand folgender Ausführungsbeispiele näher erläutert. Diese sehen eine Folge von Arbeitsschritten wie folgt vor:

### Masseherstellung:

Gezieltes Zusammenführen, Lösen bzw. Mischen von Einzelkomponenten. Als Lösemittel dient Wasser oder ein Ethanol/Wasser-Gemisch.

### Beschichtung:

- Auftragsvorrichtung:: Walzen, Streichkasten,
Düsen
- kontinuierliche Beschichtung auf:: I. strukturierte Unterlage (z.B. Teflon, Edelstahl)
II.dehäsiv ausgerüstetes Material (z.B. Papier oder Folie) mit planer Oberfläche
III. wie unter II

### Trocknung:

- zu I.: Mittels Heißluft.
- zu II.: Mittels Heißluft, wobei "wärmere und kältere" Zonen vorhanden sein müssen.
Hierdurch entsteht ein folienartiges Band, das unterschiedliche Dicken aufweist. An den wärmeren Zonen ist das Material dicker, an den kälteren ist es dünner.
Diese Zonen werden erzielt durch
a) eine durchbrochene Oberfläche, z.B. Gitter-Lochstruktur der bandförmigen Unterlage,
b) Verwendung von unterschiedlich wärmeleitenden Materialien bei der Herstellung des Transportbandes
c) dehäsiv ausgerüstetes Material, in welchem Segmente mit sich abgrenzenden wärmeleitenden Eigenschaften implantiert sind.
- Zu III.: Mittels Heißluft wie bei I. und zusätzlichem Einsatz einer strukturierten Prägewalze.

### Konfektionierung:

1. Längsschneiden in Schmalrollen.
2. Vereinzeln durch Stanzen oder Querschneiden (bei II. wird das Papier bzw. die Folie vorher entfernt).
3. Verpacken.

Weitere Einzelheiten, Merkmale und Vorteile der Erfindung ergeben sich aus der nachstehenden Erläuterung zweier in den Zeichnungen schematisch dargestellter Ausführungsbeispiele. Es zeigen:
Figur I eine schematische Darstellung im Schnitt mit Herstellung der Codierung auf einer Dickenunterschiede aufweisenden Unterlage,
Figur II im Schnitt die Herstellung einer Codierung durch Beaufschlagung der Zubereitung mit lokalen Temperaturunterschieden.

Figur I zeigt die Zubereitung (1) auf einer strukturierten Unterlage (2), welche eine Folge von Erhebungen (3) und Vertiefungen (4) aufweist. Entsprechend entstehen reziprok in der Zubereitung (1) gegenüber den Erhebungen (3) Ver-tiefungen (13) und gegenüber den Vertiefungen (4) Erhebungen (14) innerhalb der Zubereitungs-Schicht. Wärmezuführung erfolgt in einer Wärmeströmung (9), bei welcher es sich sowohl um Strahlung als auch um Konvektion handeln kann.

Figur II zeigt ein Herstellungsverfahren, bei welchem die Dickenunterschiede (13,14) in der Schicht 1 der Zubereitung beim Trocknen mit lokalen Temperaturunterschieden hergestellt werden. Dabei liegt die Zubereitungsschicht (1) auf einem folienförmigen Träger (5), beispielsweise Silikonpapier, Alufolie, Kunststofffolie oder dergleichen. Mit (6) ist ein Transportband bezeichnet, welches eine aus Stegen (7) und Durchbrüchen (8) bestehende Gitter- oder Lochstruktur aufweist. Durch Wärmezufuhr an die Stege entsprechend den Pfeilen (10) erzeugen diese im Kontakt mit der Trägerfolie (5) wärmere Bereiche und im Bereich der Durchbrüche (8) kältere Bereiche. Im Bereich der wärmeren Zonen fällt das Material bei der Trocknung dicker aus, während es in den kälteren Bereichen dünner ist. Dabei kann die Trocknung von oben her beispielsweise ebenfalls von einem Strom (9) angewärmter Luft unterstützt werden. Die Temperaturunterschiede des Transportbandes (6) können beispielsweise durch induktive Aufheizung der Stege (7) der Gitterstruktur oder durch Strahlungs- oder Kontaktheizung erzeugt werden, während die Durchbrüche (8) beispielsweise mit Luft konvektiv gekühlt werden.

Die Erfindung ist unkompliziert und erfüllt in optimaler Weise die eingangs gestellte Aufgabe.

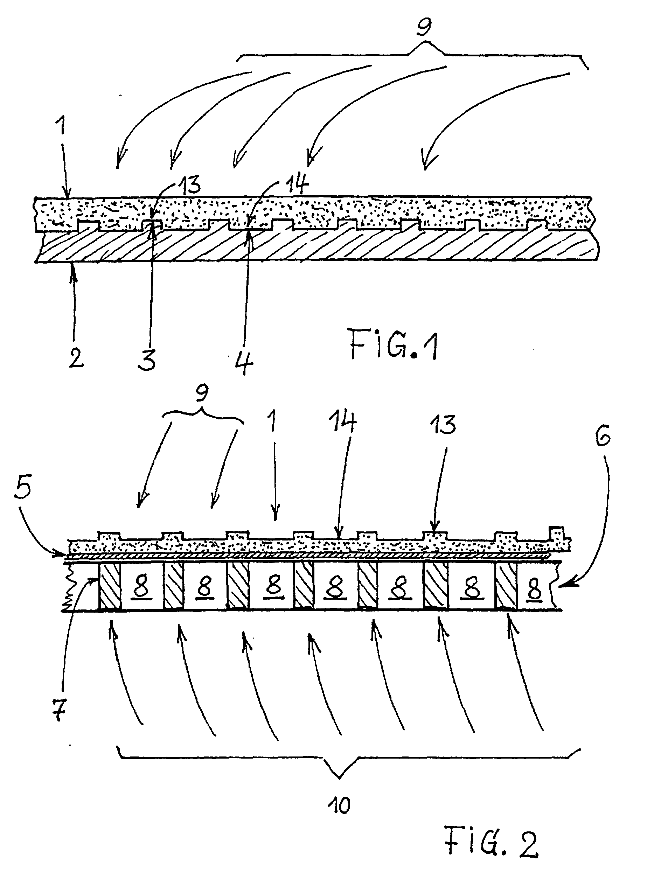

## Patentansprüche

1. Zubereitung, bestehend aus einer flächenhaften, film- oder oblatenartigen Darreichungsform mit einer Codierung durch Schrift- und/oder graphische Zeichen bzw. Muster zur Kennzeichnung der daraus bei Anwendung freisetzbaren geschmacks- oder geruchwirksamen und/oder pflegenden bzw. heilenden Substanzen, **dadurch gekennzeichnet, daß** die Codierung durch in Durchsicht erkennbare Flächenbereiche mit unterschiedlicher Dicke ausgebildet ist.

2. Zubereitung nach Anspruch 1, **dadurch gekennzeichnet, daß** die Bereiche unterschiedlicher Dicke herstellungsbedingt unter Vermeidung einer unterschiedlichen Dichte bzw. Verdichtung ausgebildet sind.

3. Zubereitung nach Anspruch 1, **dadurch gekennzeichnet. daß** die Bereiche unterschiedlicher Dicke eine unterschiedliche Dichte besitzen.

4. Verfahren zur Herstellung der Zubereitung nach einem der Ansprüche 1 oder 3, **dadurch gekennzeichnet, daß** die Bereiche unterschiedlicher Dicke unter Einwirkung von Druck ausgebildet werden.

5. Verfahren zur Herstellung der Zubereitung nach einem der Ansprüche 1 oder 2, **dadurch gekennzeichnet, daß** die Bereiche unterschiedlicher Dicke ohne Einwirkung von Druck ausgebildet werden.

6. Verfahren nach Anspruch 4 oder 5, **dadurch gekennzeichnet, daß** die Dickeunterschiede durch Beschichten einer Unterlage hergestellt werden, die eine der Codierung entsprechende Folge von Erhebungen und/oder Vertiefungen aufweist.

7. Verfahren nach Anspruch 4 oder 5, **dadurch gekennzeichnet, daß** die Dickeunterschiede durch Beaufschlagung der Zubereitung beim Trocknen mit lokalen Temperaturunterschieden hergestellt werden.

8. Verfahren nach Anspruch 4 oder 5, **dadurch gekennzeichnet, daß** die Zeichen bzw. Muster intermittierend eingebracht werden.

9. Verfahren nach Anspruch 4 oder 5, **dadurch gekennzeichnet, daß** die Zeichen bzw. Muster kontinuierlich eingebracht werden.

10. Verfahren zur Herstellung der Zubereitung nach Anspruch 1, **dadurch gekennzeichnet, daß** die Codierung der Flächenbereiche in einem plastisch verformbaren Zustand der Zubereitung erfolgt.

## Claims

1. Preparation, consisting of a sheet-like, film-like or wafer-like administration form with a coding in the form of characters and/or graphic symbols or patterns for identifying the substances affecting flavour or odour and/or the therapeutic or curative substances which can be released therefrom during use, **characterized in that** the coding is formed by surface areas of differing thickness which are visible upon looking through the preparation.

2. Preparation according to Claim 1, **characterized in that** the areas of differing thickness are formed under the production conditions while avoiding different density or compression.

3. Preparation according to claim 1, **characterized in that** the areas of differing thickness have a different density.

4. Method for producing the preparation according to one of Claims 1 to 3, **characterized in that** the areas of differing thickness are formed under the action of pressure.

5. Method for producing the preparation according to one of Claims 1 to 2, **characterized in that** the areas of differing thickness are formed without the action of pressure.

6. Method according to Claim 4 or 5, **characterized in that** the thickness differences are produced by coating of a support which has a sequence of elevations and/or depressions corresponding to the coding.

7. Method according to Claim 4 or 5, **characterized in that** the thickness differences are produced by subjecting the preparation to local temperature differences during drying.

8. Method according to Claim 4 or 5, **characterized in that** the symbols or patterns are introduced intermittently.

9. Method according to Claim 4 or 5, **characterized in that** the symbols or patterns are introduced continuously.

10. Method for producing the preparation according to Claim 1, **characterized in that** the coding of the surface areas takes place with the preparation in a plastically deformable state.

## Revendications

1. Préparation constituée d'une forme d'administration plane, du type film ou cachet avec un codage sous forme de caractères et/ou de signes graphiques resp. de dessins pour l'identification des substances actives gustatives ou olfactives et/ou préventives ou curatives susceptibles de se libérer au moment de l'emploi, **caractérisée en ce que** le codage est réalisé par des zones de surface d'épaisseur différente perceptibles en transparence.

2. Préparation selon la revendication 1, **caractérisée en ce que** les zones d'épaisseur différente, pour des raisons de fabrication, sont réalisées de manière à éviter une densité resp. une densification différente.

3. Préparation selon la revendication 1, **caractérisée en ce que** les zones d'épaisseur différente possèdent une densité différente.

4. Procédé de fabrication de la préparation selon l'une des revendications 1 ou 3, **caractérisé en ce que** les zones d'épaisseur différente sont réalisées sous l'effet de la pression.

5. Procédé de fabrication de la préparation selon l'une des revendications 1 ou 2, **caractérisé en ce que** les zones d'épaisseur différente sont réalisées en l'absence de l'effet de la pression.

6. Procédé selon la revendication 4 ou 5, **caractérisé en ce que** les différences d'épaisseur sont obtenues par revêtement d'un support comprenant une suite de bosses et/ou de creux correspondant au codage.

7. Procédé selon la revendication 4 ou 5, **caractérisé en ce que** les différences d'épaisseur sont obtenues en exposant la préparation, au moment du séchage, à des différences de température locales.

8. Procédé selon la revendication 4 ou 5, **caractérisé en ce que** les caractères resp. les dessins sont incorporés de manière intermittente.

9. Procédé selon la revendication 4 ou 5, **caractérisé en ce que** les caractères resp. les dessins sont incorporés de manière continue.

10. Procédé de fabrication de la préparation selon la revendication 1, **caractérisé en ce que** le codage des zones de surface a lieu dans un état plastiquement déformable de la préparation.
